Europäisches Patentamt

European Patent Office

Office européen des brevets

(19)

(11) Veröffentlichungsnummer: **0 183 026**

**A2**

(12) **EUROPÄISCHE PATENTANMELDUNG**

(21) Anmeldenummer: **85112930.4**

(22) Anmeldetag: **11.10.85**

(51) Int. Cl.⁴: **A 61 K 7/48**
**A 61 K 47/00**

(30) Priorität: **30.11.84 DE 3443737**

(43) Veröffentlichungstag der Anmeldung:
**04.06.86 Patentblatt 86/23**

(84) Benannte Vertragsstaaten:
**AT BE CH DE FR GB IT LI LU NL SE**

(71) Anmelder: **Giulini Chemie GmbH**
**Giulinistrasse 2**
**D-6700 Ludwigshafen/Rhein(DE)**

(72) Erfinder: **Schanz, Klaus, Dr. Dipl.-Chem.**
**In der Zeil 5**
**D-6701 Dannstadt-Schauernheim(DE)**

(72) Erfinder: **Schwind, Albert, Dr. Dipl.-Chem.**
**Heinrich Bart-Strasse 27**
**D-6702 Bad-Dürkheim(DE)**

(72) Erfinder: **Völler, Heinrich, Dr. Dipl.-Chem.**
**Kelterstrasse 34**
**D-7401 Pliezhausen(DE)**

(74) Vertreter: **Benatzky, Erika, Dr.**
**Giulinistrasse 2 Postfach 150480**
**D-6700 Ludwigshafen/Rh.(DE)**

(54) **Wenig fettige und wenig okklusive Massen zur Herstellung von Hautpflegemitteln und Salben.**

(57) Gegenstand der Erfindung sind wenig fettige, wenig okklusive Massen zur Herstellung von Hautpflegemitteln und als Träger für Arzneistoffe geeignete Massen. Sie bestehen aus einer basischen Aluminiumverbindung, von der mindestens 90 Gew.% eine Teilchengröße kleiner als 90 µm aufweisen, sowie einer Base und einem Dickungsmittel und/oder einem Konsistenzstabilisator.

EP 0 183 026 A2

- 8 -

## Wenig fettige und wenig okklusive Massen zur Herstellung von Hautpflegemitteln und Salben

Gegenstand der vorliegenden Erfindung sind wenig fettige, wenig okklusive Massen, die zur Herstellung von Hautpflegemitteln und Salben geeignet sind, sowie ein Verfahren zu ihrer Herstellung.

Massen zur Herstellung von Hautpflegemitteln und Salben sind seit langem bekannt. Sie sind heute unter der Bezeichnung Creme - bzw. Salbengrundlage auf dem Markt erhältlich und bilden den Hauptbestandteil von Hautcremes bzw. Salben. Sowohl Creme - als auch Salbengrundlagen können hinsichtlich ihrer Zusammensetzung wesentlich voneinander abweichen, wobei die Zusammensetzung jeweils vom Anwendungszweck und der gewünschten Wirkung beeinflußt wird. In Bezug auf Hautgefühl, Aussehen u.a. sind viele Grundlagen jedoch noch nicht zufriedenstellend. So haben beispielsweise wenig okklusive und nicht okklusive Hautpflegemittel und wenig okklusive und nicht okklusive Salben meistens eine ölige oder fettige Beschaffenheit, was als unangenehm empfunden wird.

In der DE-OS 26 12 330 werden auch bereits nichtfettige, okklusive Massen beschrieben, die zur Herstellung von Hautkosmetika und als Träger für Arzneistoffe geeignet sein sollen. Sie enthalten 40 bis 90 Gewichtsteile einer viskosen, gegebenenfalls eingedickten fettigen Grundlage und 60 bis 10 Gewichtsteile eines festen, salbenbildenden Pulvers, das die viskose Grundlage bei Temperaturen unter etwa $50^{\circ}C$ nicht absorbiert. Als salbenbildende Pulver werden hierbei Polyolefine oder hydratisierte Silikate eingesetzt.

2
- 7 -

Ein Nachteil der heute kommerziell erhältlichen, und zur Herstellung von Hautpflegemitteln und Salben eingesetzten Massen wird schließlich noch darin gesehen, daß sie in Abhängigkeit vom Anwendungszweck der herzustellenden Cremes und Salben in unterschiedlicher Menge und/oder unterschiedlicher Zusammensetzung eingesetzt werden müssen.

Hinsichtlich der benötigten Menge und/oder Zusammensetzung können daher die Creme- oder Salbenrezepturen erheblich voneinander abweichen. Die Dosierfehlermöglichkeit und die Lagerdiversivikationsnotwendigkeit und somit der Überwachungsaufwand können sich dadurch beträchtlich erhöhen.

Aufgabe der vorliegenden Erfindung ist es daher, Massen (Creme- und Salbengrundlagen) zu schaffen, welche die vorstehenden Nachteile nicht aufweisen, insbesondere keine fettige, auf die Haut okklusiv wirkende Beschaffenheit.

Überraschenderweise kann die gestellte Aufgabe mit Massen gelöst werden, die aus

a) 5 - 45 Gew.% einer basischen Aluminiumverbindung, von der mindestens 90 Gew.% eine Teilchengröße kleiner als 90 μm aufweisen,

b) 25 - 80 Gew.% Base

und

c) 0,5 - 50 Gew.% Dickungsmittel und/oder Konsistenzstabilisator

bestehen. Besonders gute Ergebnisse werden mit Massen erhalten, die aus 10 bis 40 Gew.% der basischen Aluminiumverbindung, 30 bis 60 Gew.% Base und

3

- 8 -

5 bis 45 Gew.% Dickungsmittel und/oder Konsistenzstabilisator bestehen. Die Massen der Erfindung sind
wenig fettig und wenig okklusiv, in vielen Fällen
sogar nicht okklusiv, obwohl die Base fettig ist.

In der vorliegenden Anmeldung werden als okklusive,
wenig okklusive bzw. nicht okklusive Masse bezeichnet:

Okklusive Masse:
Eine Masse, die die Feuchtigkeitsdampfdurchlässigkeit
durch ein natürliches Trommelfell bei bestimmten Bedingungen um mindestens größer 80 % vermindert.

Wenig okklusive Masse:
Eine Masse, die die Feuchtigkeitsdampfdurchlässigkeit
durch ein natürliches Trommelfell weniger als 20 %
vermindert.

Nicht okklusive Masse:
Eine Masse, die diese Feuchtigkeitsdampfdurchlässigkeit eine ein natürliches Trommelfell um kleiner
als 10 % vermindert.

Auf ein physikalisch/chemisches Meßverfahren zur Ermittlung von Kriterien für die Einstufung wenig fett
und nicht fett wurde verzichtet. Diese Einstufung
ist praxisnah in den Verbrauchertestpanels abgefragt
worden.

Das Verfahren zur Bestimmung der Feuchtigkeitsdampfdurchlässigkeit ist am Ende der Anmeldung beschrieben.

Als basische Aluminiumverbindungen können alle basischen Verbindungen eingesetzt werden, deren Teilchengröße mindestens zu 90 Gew.% kleiner als 90 um ist.
Derartige Verbindungen können beispielsweise getrock-

- 4 -

netes Aluminiumhydroxidgel, Aluminiumhydroxide,
Aluminiumhydroxicarbonate, auch solche des
Natriums und/oder Magnesiums und/oder Calciums sein.
Geeignet sind auch Aluminiumhydroxisulfate, Aluminiumhydroxichloride sowie Mischungen dieser Salze.

Die basischen Aluminiumverbindungen können sowohl
amorph sein als auch kristallin. Es wird angenommen,
daß die basischen Aluminiumverbindungen die fettige
Base bei erhöhter Temperatur absorbieren und dabei
in einen wenig fettigen und wenig okklusiven Zustand
versetzen, was als überraschend bezeichnet werden muß
und aus dem Stand der Technik nicht herleitbar war.

Die wenig fetten und wenig okklusiven Massen der
Erfindung zeigen einen matten Glanz und sind vom
ästhetischen Standpunkt aus gefällig. Sie sind zur
Herstellung von Hautschutzmitteln wie Emulsionen,
Cremes und Lotionen sehr gut geeignet. Sie zeigen
keine Synärese, auch ist ein Austrocknen, Abschälen
oder Springen der Massen nach Aufbringen auf die
Haut nicht zu befürchten.

Als Base können in den erfindungsgemäßen Massen Fette,
Öle, Wachse und/oder Kohlenwasserstoffe bzw. deren
Derivate enthalten sein, wobei bevorzugt pflanzliche
Öle wie Sesam-, Keim- und Kernöle, insbesondere
Jojoba- und Sojaöle, enthalten sind. Enthalten sein
können selbstverständlich auch mineralische Öle
wie Paraffinöle oder Dimethylpolysiloxane. Die Öle
können bei RT. flüssig, halbfest oder fest sein. Mit
Vorteil werden solche verwendet, deren Viskosität
bei 25°C im Bereich von 5 bis 100 m Pa·s liegt.

- 5 -

Als geeignete tierische Öle werden beispielhaft die Gemische aus acetylierten Lanolinalkoholen und Gemische aus Fettsäuren sowie Nerzöle und Schildkrötenöle genannt, die von den Firmen American Cholesterol Products bzw. Emulin Inc. auf den Markt gebracht werden.

Beispielhaft werden an dieser Stelle auch noch geeignete synthetische Öle genannt, die Gemische von Fettsäuren und/oder Fettalkoholen und/oder Alkohol-Fettsäureestern sein können, etwa Methyl-, Isopropyl- und Butylester von Fettsäuren und Acetoglycerinester bzw. Caprin- Caprylsäuretriglyceride.

Flüssige Wachse wie das natürliche Jojobaöl oder die synthetischen Nachstellungen Dynacerin 660 (R) und Cetiol 600 (R), aber auch Silikonöle wie Dimethylpolysiloxane sind als Base ebenfalls verwendbar.

Die erfindungsgemäßen Massen enthalten als weitere Komponente Verbindungen, die entweder nur als Dickungsmittel oder nur als Konsistenzstabilisator wirksam sind. Es können jedoch auch Verbindungen eingesetzt werden, die sowohl als Dickungsmittel als auch als Stabilisator wirksam sind.

Wirksame Dickungsmittel und/oder Konsistenzstabilisatoren für Basen (Öle) sind Polyolefine und gesättigte aliphatische Kohlenwasserstoffe. Sie sind bei 25°C (RT) fest und absorbieren bei erhöhter Temperatur die Öle, so daß höher viskose bis streichfähige Massen entstehen. Sie können sowohl kristallin als auch amorph sein, wobei die Gesamtbeschaffenheit (Konsistenz) der eingedickten Base durch die mengenmäßigen Anteile an kristallinen und amorphen Dickungsmittel steuerbar ist. Polyethylen, Polypro-

- 7 -

pylen, Polybutylen und Polymethylbutylen haben sich
wie Copolymerisate aus Olefinen und Vinylestern,
insbesondere aus Ethylen und Vinylacetat, in Ausführungsformen der Erfindung als zweckmäßig erwiesen.
Blockparaffine und mikrokristalline Wachse können
ebenfalls als Dickungsmittel und/oder Konsistenzstabilisatoren eingesetzt werden.

Geeignete Konsistenzstabilisatoren mit keinem oder
nur einem sehr begrenzten Verdickungseffekt sind Tenside und oberflächenaktive Substanzen mit Emulgatorwirkung und einen HLB-Wert kleiner oder gleich acht.
Genannt werden nichtionogene Tenside wie Fettalkoholethoxylate, Polypropylenglykolethoxylate, Polypropylenglykolmonofettsäureester, Fettsäuremonoglyceride,
Fettsäurereglykolpartialester, Fettsäurealkanolamide
und -diamide, Polyglycerinfettsäureester, Saccharoseester, Pentaerythrit-Partialester von Fettsäuren,
Sorbitanfettsäureester aber auch dem Cholesterin artverwandte Substanzen wie raffinierte Sojasterine
oder Phytosterine. Besonders geeignete Vertreter
sind Isotearinsäureglycerinester, Sorbitanfettsäureester, Polyoxyethylen-Cetylether sowie Mischester
aus Pentaerythrit, Fettalkoholen, Fettsäuren und Citronensäure und Propylenglykolisostearate.

Vorteilhaft ist weiterhin, daß die Massen nach der
Erfindung in an sich bekannter Weise durch homogene
Vermischung der Komponenten bei Temperaturen zwischen
0 und 150°C hergestellt werden können. Sie werden
zweckmäßigerweise unter Rühren bei Temperaturen von
20 bis 100°C miteinander vermischt, wobei die Reihenfolge der Komponentenzugabe nicht kritisch ist. In
der Regel wird man so vorgehen, daß die Base zunächst bei einer Temperatur oberhalb 50°C mit dem

- 8 -

Dickungsmittel und/oder Konsistenzstabilisator vermischt wird, gegebenenfalls unter Zugabe eines Antioxidans und eines Konservierungsmittels. Sodann wird durch Zugabe der basischen Aluminiumverbindung die fettige Base in eine wenig fettige wenig okklusive bis nicht okklusive Masse überführt. Weitere Bestandteile können der Masse vor oder nach Zugabe der basischen Aluminiumverbindung zugesetzt werden.

Die Herstellung der Massen wird wesentlich von der Viskosität der eingesetzten Base und der gewünschten Konsistenz des Endproduktes bestimmt.

Anhand der nachstehenden Beispiele und Tabellen wird der Gegenstand der Erfindung noch näher erläutert:

Beispiel für neue Massen:

| | Beispiel 1 Gew.% | Beispiel 2 Gew.% | Beispiel 3 Gew.% | Beispiel 4 Gew.% | Beispiel 5 Gew.% | Beispiel 6 Gew.% |
|---|---|---|---|---|---|---|
| basische Aluminiumverbindung | 28,00 | 28,00 | 17,95 | 17,95 | 17,5 | 35 |
| Konsistenzstabilisator | 16,00 | 24,00 | 10,25 | 15,38 | 23,3 | 20 |
| Dickungsmittel | 8,00 | 8,00 | 15,35 | 15,38 | 17,5 | 10 |
| Base | 47,80 | 39,80 | 56,25 | 51,09 | 14,50 | 34,8 |
| Antioxidans/ Konservans | 0,20 | 0,20 | 0,20 | 0,20 | 0,20 | 0,20 |

Aus den erfindungsgemäßen wenig fettigen, wenig okklusiven Massen werden beispielhaft nachstehende Fertigrezepturen für Salben, Cremes und Lotionen aufgelistet. Sie sind auf Stabilität (Tenside Detergents 21 (1984),1, S.28-30), mikrobiellen und oxidativen Verderb getestet worden.

- 8 -

Die Creme- und Lotion-Fertigrezepturen der Tabellen I bis IV wurden mit einer konstanten Mengen der erfindungsgemäßen Massen nach Beispiel 1, 2 oder 6 hergestellt. Überraschenderweise gelang es, allein durch Variation des Verhältnisses Öl-/Fettphase (B:C) zu Wasserphase für alle üblicherweise zu behandelnden Hautzustände (vereinfacht dargestellt für trockene und fette Haut) sowie alle in Frage kommenden Einsatzbereiche (Tag-, Nacht- oder Regenerationscreme) die erforderlichen Fettigkeitsgrade einzustellen.

Tabelle I

Creme- und Lotionsrezepturen in Gew.%, hergestellt mit der erfindungsgemäßen Masse

|  | Bienenwachs Nachtcreme f. extrem trock. Haut | | Wollfett Tagescreme f. extrem trock. Haut | Nachtpflegecreme f. trock. Haut |
|  | A | B |  |  |
| --- | --- | --- | --- | --- |
| A) Masse nach Beisp. 1+2 | 12,5 | 12,5 | 12,5 | 12,5 |
| B) Öl-/Fett-Phase |  |  |  |  |
| Acetulan | – | 1,5 | – | – |
| Avocadoöl | 0,5 | 0,5 | 0,5 | 0,5 |
| Biocornöl | 1,5 | – | – | 1,0 |
| Calendalaöl | 0,5 | 0,5 | 0,5 | – |
| ß-Carotin | 0,002 | 0,002 | 0,002 | – |
| Eusolex 8021 | – | – | 1,0 | – |
| Jojoba-Substitut | – | – | 1,0 | 1,0 |
| Bienenwachs | 2,0 | 2,0 | 1,0 | – |
| Maiskeimöl | 3,0 | 3,0 | 3,0 | 3,0 |
| Microwachs HP 67 | 2,0 | 2,0 | 3,0 | 2,0 |
| Paraffinöl | 10,0 | 10,0 | 8,0 | 10,0 |

- 14 -

| | | | | |
|---|---|---|---|---|
| Lanolin | 0,5 | 0,5 | 2,0 | – |
| Sojaöl raff. | 4,5 | 4,5 | 4,5 | 5,5 |
| Sonnenblumenöl | 0,5 | 0,5 | 0,5 | – |
| Vitamin A-Palmitat | 0,5 | 0,5 | 0,5 | 0,5 |
| Fette/Öle | 38,002 | 38,002 | 38,002 | 36,000 |

C) Wasserphase

| | | | | |
|---|---|---|---|---|
| Wasser demineral. | 56,148 | 56,148 | 56,048 | 57,250 |
| Zinnkrautextrakt | – | – | – | 0,5 |
| Schafsgarbeextrakt | – | – | – | 0,5 |
| Allantoin | 0,5 | 0,5 | 0,5 | 0,5 |
| Gilustab | 1,0 | 1,0 | 1,0 | 1,0 |
| Vitamin E acetat | – | – | – | 0,50 |
| Hamamelis | 0,5 | 0,5 | 0,5 | 0,5 |
| Joghurt-Pulver | 0,1 | 0,1 | 0,1 | – |
| Sorbitol | 3,0 | 3,0 | 3,0 | 3,0 |
| Titriplex III | – | – | 0,1 | – |
| Kollagen T | 0,5 | 0,5 | 0,5 | – |
| Parfüm | 0,25 | 0,25 | 0,25 | 0,25 |
| Wasser-System | 61,998 | 61,998 | 61,998 | 64,00 |

Tabelle II

Creme- und Lotionsrezepturen in Gew.%,

hergestellt mit der erfindungsgemäßen Masse

| | Tagcreme trock. Haut m.UV A+B Schutz | Nachtcreme norm.-fette Haut | Tagcreme norm-fette Haut | Tagcreme norm.-fette Haut UV A+B-Schutz |
|---|---|---|---|---|
| A)<br>Masse nach Beisp. 1+2 | 12,5 | 12,5 | 12,5 | 12,5 |

B)
Öl-/Fett-
Phase

| | | | | |
|---|---|---|---|---|
| Azulen | 0,002 | – | – | – |
| Avocadoöl | 0,5 | 0,5 | 0,5 | 0,5 |
| Biocornöl | – | 5,0 | – | – |
| Calendaöl | 0,5 | – | 0,5 | 0,5 |
| ß-Carotin | – | – | – | 0,002 |
| Eusolex 8021 | 1,0 | – | – | 0,5 |
| Jojoba-Substitut | 1,0 | 1,0 | 2,0 | 2,0 |
| Bienenwachs | 0,5 | – | – | – |
| Maiskeimöl | 3,0 | 3,0 | 3,0 | 3,0 |
| Microwachs HP 67 | 2,0 | 2,0 | 2,0 | 2,0 |
| Paraffinöl | 10,0 | 5 | 3,0 | 2,5 |
| Sojaöl raff. | 3,0 | 0,85 | 0,85 | 0,50 |
| Sonnenblumenöl | 0,5 | – | 0,5 | 0,5 |
| Vitamin A-Palmitat | 0,5 | 0,5 | 0,5 | 0,5 |
| **Fette/Öle** | 35,002 | 30,35 | 25,35 | 25,002 |

C)
Wasserphase

| | | | | |
|---|---|---|---|---|
| Wasser demineral. | 58,648 | 63,20 | 69,40 | 69,498 |
| Gurkensaft | – | 0,2 | – | – |
| Allantoin | 0,5 | 0,5 | 0,5 | 0,5 |
| Gilustab® | 1,0 | 1,0 | 1,0 | 1,0 |
| Thymianextrakt | – | 0,5 | – | – |
| Hamamelis | 0,5 | 0,5 | 0,5 | 0,5 |
| Kamilleextrakt | 0,5 | – | – | – |
| Sorbitol | 3,0 | 3,0 | 3,0 | 3,0 |
| Titriplex III | 0,1 | – | – | 0,25 |
| Kollagen T | 0,5 | 0,5 | – | – |
| Parfüm | 0,25 | 0,25 | 0,25 | 0,25 |
| **Wasser-System** | 64,998 | 69,65 | 74,65 | 74,998 |

Tabelle III

Creme- und Lotionsrezepturen in Gew.%
hergestellt mit der erfindungsgemäßen Masse

| | Tagcreme norm.-trock. Haut | Nachtcreme norm.-trock. Haut | Skin Moist Lotion mit UV A+B Schutz |
|---|---|---|---|
| A) Masse nach Beisp.1+2 | 12,5 | 12,5 | 12,5 |
| B) Öl-/Fett-Phase | | | |
| Arlacel 989 | - | - | 1,5 |
| Avocadoöl | 6 | 0,5 | 1 |
| Biocornöl | - | 0,5 | 1 |
| Calendalaöl | - | - | 2 |
| Eusolex 8021 | - | - | 0,5 |
| Isopropylmyristat | - | - | 3,15 |
| Jojoba-Substitut | 1,0 | 1,0 | 1,0 |
| Lanette C | - | 0,5 | - |
| Maiskeimöl | 3,0 | 3,0 | - |
| Microwachs HP 67 | 2,0 | 2,0 | 1,0 |
| Paraffinöl | 5 | 10,0 | - |
| Sojaöl raff. | 0,85 | 1,00 | - |
| Sonnenblumenöl | - | 0,5 | - |
| Vitam A-Palmitat | - | - | 0,5 |
| Fette/Öle | 30,35 | 31,50 | 24,15 |
| C) Wasserphase | | | |
| Wasser demineral. | 64,40 | 63,25 | 65,10 |
| Schafsgarbe-extrakt | - | - | 0,5 |
| Allantoin | 0,5 | 0,5 | 0,2 |
| Johanniskrt. extrakt | - | - | 0,5 |

- 12 -

| | | | |
|---|---|---|---|
| Gilustab(R) | 1,0 | 1,0 | 1,0 |
| Vitamin E acetat | - | - | 0,5 |
| Hamamelis | 0,5 | 0,5 | 0,5 |
| Kamille-extrakt | - | - | 0,5 |
| Propylen-glykol | - | - | 4 |
| Sorbitol | 3,0 | 3,0 | 2,0 |
| Titriplex III | - | - | 0,25 |
| Kollagen T | - | - | 0,5 |
| Orangen-essenz | - | - | 0,15 |
| Parfüm | 0,25 | 0,25 | 0,15 |
| Wasser-System | 69,65 | 68,50 | 75,85 |

Tabelle IV

Creme- und Lotionsrezepturen in Gew.%

hergestellt mit der erfindungsgemäßen Masse

| | Vitamin Regene-rations-creme | Moisture Sonnen-creme SF 6 | Moistu-rizing Sonnen-lotion SF 4 |
|---|---|---|---|
| A)<br>Masse nach Beisp.1+2 | 12,5 | 12,5 | 12,5 |
| B)<br>Öl-/Fett-Phase | | | |
| Arlacel 989 | - | - | 1,5 |
| Nifalan MG | 1,0 | - | - |
| Avocadoöl | 0,5 | 0,5 | 1 |
| Biocornöl | 1,0 | - | 1 |
| ß-Carotin | 0,002 | - | - |
| Eusolex 6300 | - | 2 | 0,5 |
| Isopropyl-myristat | - | - | 3,15 |
| Jojoba-Substitut | 1,0 | 1,0 | - |
| Bienenwachs | 2,0 | - | - |
| Lanette C | 0,5 | - | - |
| Maiskeimöl | 3,0 | 3,0 | - |

13
- 18 -

| | | | |
|---|---|---|---|
| Microwachs HP 67 | 2,0 | 4,0 | 1,0 |
| Paraffinöl | 5,0 | 8 | - |
| Sojaöl raff. | 0,8 | 0,8 | 0,8 |
| Sonnenblumenöl | 0,5 | 0,5 | - |
| Vitamin A-Palmitat | 1 | - | 0,5 |
| **Fette/Öle** | 30,802 | 32,30 | 21,95 |

C)
__Wasserphase__

| | | | |
|---|---|---|---|
| Wasser demineral. | 61,198 | 59,45 | 69,15 |
| Gurkensaft | - | - | 0,2 |
| Schafsgarbeextrakt | 0,5 | - | - |
| Allantoin | 0,25 | 0,5 | 0,2 |
| Johanniskrt. extrakt | 0,5 | - | - |
| Gilustab | 1,0 | 1,0 | 1,0 |
| Vitamin E acetat | 1,0 | - | - |
| D-Panthenol | 0,5 | - | - |
| Hamamelis | 0,5 | 0,5 | - |
| Propylenglykol | - | - | 4 |
| Eusolex 232 | - | 2 | 0,5 |
| Sorbitol | 3,0 | 3,0 | 2,0 |
| Triäthanolamin conc. | - | 1 | 0.25 |
| Kollagen T | 0,5 | - | 0,5 |
| Parfüm | 0,25 | 0,25 | 0,25 |
| **Wasser-System** | 69,198 | 67,70 | 78,05 |

14
- 19 -

Die Salben in den nachstehenden Beispielen 7 bis 10
wurden durch Vermischen der erfindungsgemäßen Masse
nach Beispiel 5 mit Ölen und Wachsen bei 70°C und
anschließende Abkühlung hergestellt.

Beispiel 7   (Schwefelhaltige Salbe)
60 Gew.%   Masse nach Beispiel 5
15 Gew.%   kolloidaler Schwefel
25 Gew.%   Öle und Wachse

Beispiel 8   (Zinkoxidhaltige Salbe)
70 Gew.%   Masse nach Beispiel 5
10 Gew.%   Zinkoxid
20 Gew.%   Öle und Wachse

Beispiel 9   (Na - salicylathaltige Salbe)
75 Gew.%   Masse nach Beispiel 5
 5 Gew.%   Natriumsalicylat
25 Gew.%·  Öle und Wachse

Beispiel 10 (Borsäuresalbe)
80 Gew.%   Masse nach Beispiel 5
 5 Gew.%   Borsäure
15 Gew.%   Öle und Wachse

Die Cremes, Lotionen und Salben gemäß vorstehenden
Tabellen und Beispielen wurden hinsichtlich ihrer
Okklusivität nach einem eigens hierfür entwickelten
Verfahren getestet. Nach dieser Methode wird zunächst
der Wasserverlust bestimmt, der beim Durchtritt durch
eine natürliches Trommelfell zu beobachten ist. Anschließend wird auf das Trommelfell die okkludierend
wirkende Creme oder Lotion aufgebracht, und ebenfalls
der Wasserverlust gemessen, der beim Durchtritt durch
das behandelte Trommelfell auftritt. Der Wasserverlust

- 15 -

wird als Feuchtigkeitsdampfdurchlässigkeit (MVT) angegeben und nach folgender Gleichung berechnet:

$$MVT = \frac{W}{AT}$$

In dieser Gleichung bedeutet W das Gewicht des verlorenen
Wasser in mg, A die für den Dampfdurchlaß zur Verfügung stehende Fläche in $cm^2$ und T die Zeit in Stunden,
während der die Dampfdurchlässigkeit bestimmt wird.

Anschließend wird die prozentuale Okklusivität (% Occ)
nach folgender Gleichung berechnet:

$$\%Occ = 100 \cdot 1 - \frac{MVT_s}{MVT_c}$$

In dieser Gleichung bedeutet $MVT_s$ und $MVT_c$ die Feuchtigkeitsdampfdurchlässigkeit durch das okkludierte Substrat
(Creme, Salbe ect.) bzw. durch das Kontrollträgermaterial (Trommelfell).

Dieses Testverfahren zur Messung der Feuchtigkeitsdampfdurchlässigkeit läßt sich leicht durchführen.
Der MVT-Kontrollwert wird gemessen, in dem man eine
bekannte Menge Wasser (220 bis 300 g) in ein 0,35 Ltr.
fassendes Gefäß gibt. Die Oberfläche des Gefäßes ist
mit einem natürlichen Trommelfell verklebt. Die Vorrichtung wird samt ihrem Inhalt 48 bis 77 Stunden in
eine Testkammer mit einer relativen Feuchtigkeit von
51 % und einer Temperatur von 70°C gestellt. Ein Luftstrom von 16 bis 30 m/min. wird durch die Kammer geleitet. Anschließend wird gewogen. Der Wasserverlust
zwischen Anfangs- und Endwägung wird berechnet und
ergibt den $MVT_c$- Wert. Auch

der MVT$_s$-Wert der zu untersuchenden Cremes wird nach dem vorgenannten Verfahren gemessen, indem man 0,4 g der zu untersuchenden Creme auf das Trommelfellträgermaterial mit der Fingerspitze aufträgt. Anschließend werden die 0,35 1 Gefäße mittels Injektionsspritze durch das Trommelfell wieder mit Wasser aufgefüllt, ausgewogen und erneut 48 bis 77 Stunden in die Testkammer (mit gleicher relativer Luftfeuchtigkeit von 54 % und gleicher Temperatur von 70$^o$C und Luftstrom von 16 bis 30 m/min.) gestellt. Die Okklusivität wird nach dem vorbeschriebenen Verfahren und der angegebenen Formel berechnet.

Bei allen MVT-Messungen wird als Substrat ein natürliches Trommelfell Standard-Trägermaterial verwendet.

Wie vorstehende Rezepturen und weitere Untersuchungen gezeigt haben, sind Hautpflegemittel auf Basis der neuen Massen dann optimal, wenn sie

$$5 - 30 \text{ Gew.\%} \quad \text{der Masse}$$
$$7 - 30 \text{ Gew.\%} \quad \text{Fett/Ölphase}$$
$$\text{und} \quad 50 - 80 \text{ Gew.\%} \quad \text{Wasserphase}$$

enthalten.

Salben enthalten 50 bis 80 Gew.% der neuen Masse, 20 bis 50 Gew.% Fett/Ölphase (Öle und Wachse) sowie Wirkstoffe.

- 22 -

Tabelle V zur Bestimmung prozentualer Okklusivität und zwar der Creme aus Tabelle II (Tagcreme normal – fette Haut UV A+B Schutz)

| $W_c$ (mg) | $T_c$ (h) | $MVT_c \cdot 10^{-2}$ | % Occ. |
|---|---|---|---|
| 53,0 | 72 | 2,09 | 100-98    =   2,0 |
| 63,2 | 72 | 2,49 | 100-95,2 =   4,8 |
| 50,0 | 72 | 1,97 | 100-92,4 =   7,6 |
| 55,1 | 72 | 2,17 | 100-88,0 = 12,0 |
| 46,1 | 48 | 2,72 | 100-93,4 =   6,6 |
| 42,5 | 48 | 2,51 | 100-88,8 = 11,2 |

| $W_s$ (mg) | $T_s$ (h) | $MVT_s \cdot 10^{-2}$ |
|---|---|---|
| 56,0 | 77 | 2,06 |
| 64,4 | 77 | 2,37 |
| 46,1 | 72 | 1,82 |
| 48,5 | 72 | 1,91 |
| 43,1 | 48 | 2,54 |
| 37,7 | 48 | 2,23 |

Die gemessenen Werte zeigen, daß die prozentuale Okklusivität niedrig liegt, im arithmetischen Mittel bei 7,4 %.

<u>Patentansprüche</u>

1) Wenig fettige, wenig okklusive, zur Herstellung von Hautpflegemitteln und als Träger für Arzneistoffe geeignete Massen, bestehend aus

    a) 5 - 45 Gew.% einer basischen Aluminiumverbindung, von der mindestens 90 Gew.% eine Teil- chengröße kleiner als 90 µm aufweisen,

    b) 25 - 80 Gew.% Base

und

    c) 0,5 - 50 Gew.% Dickungsmittel und/oder Konsistenzstabilisator.

2) Massen nach Anspruch 1, bestehend aus

    a) 10 - 40 Gew.% einer basischen Aluminiumverbindung, von der mindestens 90 Gew.% eine Teil- chengröße kleiner als 90 µm aufweisen,

    b) 30 - 60 Gew.% Base

und

    c) 5 - 45 Gew.% Dickungsmittel und/oder Konsistenzstabilisator.

3) Massen nach den Ansprüchen 1 und 2, dadurch gekennzeichnet, daß sie als basische Aluminiumverbindung ein Aluminiumhydroxid, insbesondere Aluminiumhydroxidgel, enthalten.

4) Massen nach den Ansprüchen 1 und 2, dadurch gekennzeichnet, daß sie als basische Aluminium- verbindung ein Aluminiumhydroxi-carbonat ent- halten.

5) Massen nach Anspruch 4, dadurch gekennzeichnet, daß sie als basische Aluminiumverbindung ein Aluminiumhydroxi-carbonat des Natriums und/oder Magnesiums und/oder Calciums enthalten.

6) Massen nach den Ansprüchen 1 und 2, dadurch gekennzeichnet, daß sie als basische Aluminiumverbindung Aluminiumhydroxisulfat enthalten.

7) Massen nach den Ansprüchen 1 und 2, dadurch gekennzeichnet, daß sie als basische Aluminiumverbindung ein Aluminiumhydroxichlorid enthalten.

8) Massen nach den Ansprüchen 1 und 2, dadurch gekennzeichnet, daß sie als basische Aluminiumverbindung mindestens zwei der Aluminiumverbindungen gemäß den Ansprüchen 3 bis 7 enthalten.

9) Massen nach den Ansprüchen 1 bis 8, dadurch gekennzeichnet, daß sie als Base Fette, Öle, Wachse und/oder Kohlenwasserstoffderivate enthalten.

10) Massen nach Anspruch 9, dadurch gekennzeichnet, daß sie pflanzliche Öle wie Keim- und Kernöle, Sesam- und Jojobaöle enthalten, insbesondere Sojaöle.

11) Massen nach Anspruch 9, dadurch gekennzeichnet, daß sie Paraffinöle und/oder Siliconöle enthalten.

12) Massen nach den Ansprüchen 1 bis 11, dadurch gekennzeichnet, daß sie als Dickungsmittel und/oder Konsistenzstabilisator Polyolefine, wie Polyethylen, Polypropylen, Polybutylen oder Polymethylbutylen, enthalten.

13) Massen nach den Ansprüchen 1 bis 11, dadurch gekennzeichnet, daß sie als Dickungsmittel und/oder Konsistenzstabilisator Naturwachse wie Bienenwachs, Hartparaffin und/oder mikrokristallines Wachs enthalten.

14) Massen nach den Ansprüchen 1 bis 11, dadurch gekennzeichnet, daß sie als Dickungsmittel und/oder Konsistenzstabilisator ein Olefin - Acetat - Copolymerisat enthalten, beispielsweise ein Ethylen - Vinylacetat - Copolymerisat.

15) Massen nach den Ansprüchen 1 bis 11, dadurch gekennzeichnet, daß sie als Dickungsmittel und/oder Konsistenzstabilisator Polyacrylsäure enthalten.

16) Massen nach den Ansprüchen 1 bis 15, dadurch gekennzeichnet, daß sie als Konsistenzstabilisator ein Tensid mit Emulgatorwirkung enthalten, insbesondere nichtionogene Tenside wie Fettalkohol- und Polypropylenglykolethoxylate, Fettsäureglyceride und Fettsäurealkanolamide.

17) Massen nach den Ansprüchen 1 bis 15, dadurch gekennzeichnet, daß sie als Konsistenzstabilisator Isostearinsäureglycerinester enthalten.

18) Massen nach den Ansprüchen 1 bis 15, dadurch gekennzeichnet, daß sie als Konsistenzstabilisator Sorbitfettsäureester enthalten.

19) Massen nach den Ansprüchen 1 bis 15, dadurch gekennzeichnet, daß sie als Konsistenzstabilisator Polyoxyethylencethylether enthalten.

– 4 –

20) Massen nach den Ansprüchen 1 bis 15, dadurch gekennzeichnet, daß sie als Konsistenzstabilisator Mischester aus Pentaerythrit, Fettalkohol, Fettsäure und Citronensäure enthalten.

21) Massen nach den Ansprüchen 1 bis 15, dadurch gekennzeichnet, daß sie als Konsistenzstabilisator Propylenglykolstearat enthalten.

22) Massen nach den Ansprüchen 1 bis 21, dadurch gekennzeichnet, daß sie zusätzlich Hilfsstoffe enthalten, insbesondere Antioxidantien und Konservierungsstoffe.

23) Verfahren zur Herstellung der Massen nach den Ansprüchen 1 bis 22, dadurch gekennzeichnet, daß die Komponenten bei Temperaturen von 0 bis 150°C homogen vermischt werden.

24) Verwendung der in den Ansprüchen 1 bis 22 geschützten Massen in wenig fettigen, wenig okklusiven Hautpflegemitteln, insbesondere Emulsionen, Cremes und Lotionen.

25) Verwendung der Massen gemäß Anspruch 24 in Hautpflegemitteln, bestehend aus

5 – 30 Gew.% wenig fettige, wenig okklusive Masse

7 – 30 Gew.% Fett/Ölphase

und

50 – 80 Gew.% Wasserphase.

26) Verwendung der in den Ansprüchen 1 bis 22
geschützten Massen als Träger für Arzneistoffe
in Salben.

27) Verwendung der Massen gemäß Anspruch 26 in
Salben, bestehend aus

50 - 80 Gew.% wenig fettige, wenig okklusive
Masse
und
20 - 50 Gew.% Fett/Ölphase.